(19) Europäisches Patentamt
European Patent Office
Office européen des brevets

(11) Publication number: **0 057 473 A2**

# (12) EUROPEAN PATENT APPLICATION

(21) Application number: **82101502.1**

(51) Int. Cl.³: **C 07 D 213/64**, C 07 D 213/70

(22) Date of filing: **30.06.78**

(30) Priority: **22.07.77 US 817943**

(43) Date of publication of application: **11.08.82**
**Bulletin 82/32**

(84) Designated Contracting States: **BE DE FR GB NL SE**

(60) Publication number of the earlier application in accordance with Art. 76 EPC: **0017767**

(71) Applicant: **THE DOW CHEMICAL COMPANY, Dow Center 2030 Abbott Road Post Office Box 1967, Midland Michigan 48640 (US)**

(72) Inventor: **Johnston, Howard, Las Lomas Way, Walnut Creeek California (US)**
Inventor: **Heitz Troxell, Lillian, 1715 Hillcrest, Antioch California (US)**

(74) Representative: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

## (54) A process for preparing trifluoromethylpyridyl (oxy/thio) phenoxypropionic compounds being especially active as herbicides.

(57) A process for preparing trifluoromethylpyridyl(oxy/thio) phenoxy propionic compounds especially active as herbicides for the control of grassy or graminaceous weeds, corresponding to the formula I

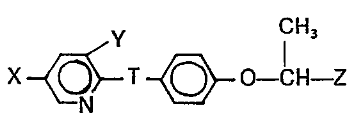

(I)

weherein
T is oxygen or sulfur;
X is Cl, Br or $CF_3$;
Y is H, Cl, Br or $CF_3$, provided at least one of X and Y is $CF_3$;
Z is -C-OR, $-CNR_1R_2$, -C-O⊖M⊕, -CN, $-CH_2OR_3$, $-CH_2OCR_4$ or $-CSR_5$;
M is $⊕NHR_1R_1R_1$, Na, K, Mg or Ca;
R is H, $C_{1-8}$ alkyl, benzyl, chlorobenzyl, or $C_{3-6}$ alkoxyalkyl;
$R_1$ is H, $C_{1-4}$ alkyl, or $C_{2-3}$ hydroxyalkyl;
$R_2$ is $H_1$ or $-OCH_3$;
$R_3$ is H or $C_{1-4}$ alkyl;
$R_4$ is $C_{1-7}$ alkyl; and
$R_5$ is $C_{1-4}$ alkyl, as disclosed in the published European Patent Application No. 0 000 483, comprising the using as an intermediate of a trifluoromethylpyridyl(oxy/thio)phenol compound corresponding to the formula II

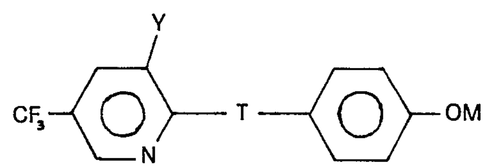

(II)

wherein
T is oxygen or sulfur; and
Y is H or Cl, and M is H or a cation of a caustic solution.

EP 0 057 473 A2

The invention relates to a process for preparing Trifluoromethylpyridyl(oxy/thio)phenoxypropionic compounds as disclosed in the published European Patent Application No. 0 000 483, which are usable for the control of undesired plant growth and which are especially active as herbicides for the control of grassy or graminaceous weeds.

The subject matter of the present invention comprises a process for perparing trifluoromethylpyridyl(oxy/thio) phenoxypropionic compounds especially active as herbicides for the control of grassy or graminaceous weeds corresponding to the formula I

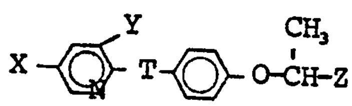

wherein T is oxygen or sulfur;

X is Cl, Br or $CF_3$;

Y is H, Cl, Br or $CF_3$, provided at least one of X and Y is $CF_3$;

Z is -C-OR, $-CNR_1R_2$, $-C-O^{\ominus}M^{\oplus}$, -CN, $-CH_2OR_3$, $-CH_2OCR_4$, or $-CSR_5$;

M is $^{\oplus}NHR_1R_1R_1$, Na, K, Mg, or Ca;

R is H, $C_{1-8}$ alkyl, benzyl, chlorobenzyl, or $C_{3-6}$ alkoxyalkyl;

$R_1$ is H, $C_{1-4}$ alkyl, or $C_{2-3}$ hydroxyalkyl;

$R_2$ is $R_1$ or $-OCH_3$;

$R_3$ is H or $C_{1-4}$ alkyl;

$R_4$ is $C_{1-7}$ alkyl; and

$R_5$ is $C_{1-4}$ alkyl; as disclosed in the published European Patent Application No. 0 000 483

c h a r a c t e r i z e d  b y  using as an intermediate a trifluoromethylpyridyl(oxy/thio)phenol compound corresponding to the formula II

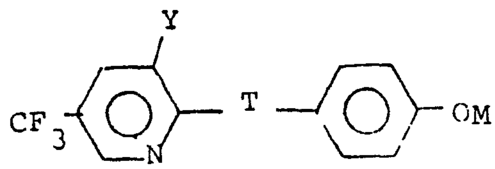

(II)

wherein T is oxygen or sulfur; and

Y is H or Cl, and M is H or a cation of a caustic solution.

According to a prefered embodiment of said process the following compounds of the above general formula II are used as an intermediate:
4-(5-trifluoromethyl-2-pyridyloxy)phenol,
4-(5-trifluoromethyl-2-pyridylthio)phenol,
4-(3-chloro-5-trifluoromethyl-2-pyridylthio)phenol or
4-(3-chloro-5-trifluoromethyl-2-pyridyloxy)phenol.

According to a further prefered embodiment of the invention the process is carried out by reacting said intermediate of general formula II with a propanoic ester and optionally converting the product obtained to the desired derivative of the above general formula I.

To accomplish the preparation of the trifluoromethylpyridyl(oxy/thio)phenoxypropionic compounds of the above-noted general formula I, which are suited for the control of perennial grassy weeds resulting in control of a broader spectrum of such weeds than the related prior art compounds while exhibiting a higher level of activity or control at like dosage rates, the 4-(trifluormethyl)-substituted-2-pyridyloxy)phenol or 4-(trifluormethyl-substituted-2-pyridylthio)phenol of the above general formula II is dissolved in a solvent

such as dimethylsulfoxide, anhydrous powdered sodium hydroxide is added thereto and reacted therewith for a few minutes at about 75-85°C. Then a propanoic ester, such as the ethylester of 2-bromopropanoic acid is added to the reaction mixture and stirred for a time, such at about half an hour, at approximately 100°C or up to about two hours in the case of the sulfur bridged compound. The reaction mixture is then allowed to cool and poured over ice or simple into cold water whereupon an oily layer separates which can be recovered by taking up in a water-immiscible solvent and subsequently stripping the solvent off leaving an oily product. The product so obtained will be the alkylester of the propanoic acid compound. Fora more detailed description reference is made to the European Patent Application No. 7810291, published under No. 0 000483, from which the parent case of the present application, i.e. the European Patent Application No. 80101361.6 (published under No. oo17767) is a Divisional.

The intermediates of the general formula II used in the process of the present application are specifically disclosed in the European Patent Application No. 80101361.6 (publication No. 0017767), from which the present application is a Divisional. For preparing said compounds of the general formula II, a salt, e.g. the sodium salt, of 4-methoxyphenol, or of 4-mercaptophenol, is dissolved in a solvent such as dimethyl sulfoxide and the requisite trifluoromethyl-substituted 2-chloropyridine is added to the solution of the methoxy phenol and reacted in the presence of aqueous sodium hydroxide at a temperature in the range of at least 50, preferably about 70 to 150°C and over a time interval of about 30 to 45 minutes. The reaction mixture is the cooled and poured over ice. The solid product is filtered off, washed

with water, taken up in a solvent mixture and reprecipitated therefrom. The methoxy group, if present, is then cleaved off the phenyl ring by refluxing the compound in 48 % by weight HBr for about an hour and after purification, precipitated from acidic solution and recovered, as by filtration, and dried. In carrying out the several reactions of this process, the reactants are usually mixed with a carrier medium, such as, for example, methyl ethyl ketone, methyl isobutyl ketone or an aprotic polar solvent such as dimethylformamide, dimethylacetamide, dimethylsulfoxide, N-methylpyrrolidone, hexamethylphosphoramide, or sulfolane. The condensation is generally carried out at a temperature of at least 50°C, preferably about 70 to about 150°C and during a reaction period of about 1 to about 20 hours, preferably about 1 to about 10 hours. The dealkylation step, where employed, is carried out using as a suitable dealkylation agent, a hydro acid such as hydrobromic acid or hydriodic acid employed as a concentrated aqueous solution of about 40 to about 60 percent by weight concentration. Reaction is carried out at reflux temperature which usually falls in the range of about 75 to about 150°C but preferably is about 100°C to 140°C. The dealkylation reaction is generally completed in about 1 to about 10 hours.

The following production examples A, B , C and D cover the preparation of the intermediates of the above general formula II used according to the process of the present invention.

Production Example A

A solution of the sodium salt of 4-methoxyphenol was prepared by dissolving the methoxyphenol (7.45 g; o.o6 mole) in 45 ml of dimethylsulfoxide and adding a solution of sodium hydroxide (2.4 g; o.o6 mole) in 7 ml of water. A solution of 2-chloro-5-(trifluoromethyl)pyridine (9.o g; o.o5 mole) in 4o ml of dimethylsulfoxide was then added to the above sodium phenate solution over an 11 minute period. During the addition, the temperature rose to about 8o$^{o}$C, and then the reaction mixture was heated to 124$^{o}$C over 26 minutes and the temperature maintained for 15 minutes. At the end of this time, the reaction mixture was cooled to 75$^{o}$C and poured over ice. The solid product was collected on a filter, washed, and taken up in a toluene-hexane mixture. This solution on cooling yielded 9.7 grams of solid product having a melting temperature of 49.5 - 5o.5$^{o}$C and having a composition of 58.o2 % carbon; 3.86 ( hydrogen; and 5.22 % nitrogen. The theoretical composition is 57.99 % carbon; 3.74 % hydrogen; and 5.2o % nitrogen, confirming the product to be 5-(trifluoromethyl)-2-(4-methoxyphenoxy)pyridine.

The 5-(trifluoromethyl)-2-(4-methoxyphenoxy)-pyridine (1o.95 g; o.o4o7 mole) was refluxed with 5o ml of 48 percent by weight aqueous hydrobromic acid solution for one hour. At the end of this time, the reaction mixture was cooled, poured over ice and the separated solids collected on a filter. The product was purified by taking it up in dilute caustic solution, extracting the solution with chloroform to remove unreacted starting material and then acidifying the solution to precipitate free phenol. The dried crystalline phenol product had a melting temperature of 89-91$^{o}$C and was found to

contain 56.21 % carbon; 3.27 % hydrogen; and 5.44 % nitrogen. The theoretical composition of 4-(5-trifluoromethyl-2-pyridyloxy)phenol is 56.48 % carbon; 3.16 % hydrogen; and 5.49 % nitrogen.

Production Example B

4-Mercaptophenol (7.6 g, o.o6 mole) was dissolved in 7o ml of dimethyl sulfoxide, and a solution of sodium hydroxide (2.4 g, o.6 mole) in 3.o ml of water was added. The mixture was warmed to 5o$^{o}$ and stirred under nitrogen for 1o minutes to form the sodium thiophenate salt. A solution of 2-chloro-5-(trifluoromethyl)pyridine (1o.9 g, o.o6 mole) in 6o ml of dimethylsulfoxide was then added all at once. The mixture was heated to 1oo$^{o}$ and held there for 1.5 hours. At the end of this time it was poured into 5oo ml of cold water. An emulsion formed; therefore, 6o ml of a saturated solution of ammonium chloride was added. The product precipitated as a sticky solid. The aqueous layer was decanted, the solid washed with more water, then taken up in hot heptane, dried with solid sodium sulfate and decolorized with charcoal. In the filtrate, a white solid product precipitated and was separated and found to have a melting temperature of 89-93$^{o}$C.

Production Example C

4-Mercaptophenol (6.4 g, o.o51 mole) was dissolved in 7o ml of dimethylsulfoxide and a solution of sodium hydroxide (2.o4 g, o,o51 mole) in 3.o ml of water was added. The mixture was warmed to about 5o$^{o}$ and stirred under nitrogen for 1o minutes to form the sodium thiophenate salt. A solution of 2,3-dichloro-5-(trifluoromethyl)pyridine (11.o g, o.o51 mole) in 6o ml of dime-

BAD ORIGINAL

thylsulfoxide was next added all at once. The mixture was then heated at 95-1oo° for 2.5 hours, then poured into 5oo ml of cold water and allowed to stand for 45 minutes. The solid was then collected on a filter, washed and taken up in about one liter of boiling hexane. The product precipitated on cooling as a white solid melting at 94-96°C. The so-prepared compound is 4-[3-chloro-5-trifluoromethyl-2-pyridylthio]phenol (11.o g, o.o36 mole).

Production Example D

2,3-Dichloro-5-trifluoromethylpyridine was dissolved in 9o ml DMSO and 1o.8 g hydroquinone added. The reactants were maintained under nitrogen. Heated to 8o°C and slowly added 9.9 ml 5o % NaOH - 2 hours. Heated to 1oo°C for 1.5 hours and poured into ice. Then added 19 g concentrated HCl. Decanted, added more water and 5 g HCl to keep acidic. Gummy material was taken up in heptane, the DMSO-solution deodorized with charcoal and chilled. The filtered product was dried and analyzed.

| | C | H | N | Cl |
|---|---|---|---|---|
| calculated: | 49.76 | 2.44 | 4.84 | 12.24 |
| found: | 48.74 | 2.76 | 4.55 | 12.o5 |

The following example illustrate the present invention:

Example 1

A solution of the sodium salt of 4-methoxyphenol was prepared by dissolving the methoxyphenol (7.45 g; 0.06 mole) in 45 ml of dimethylsulfoxide and adding a solution of sodium hydroxide (2.4 g; 0.06 mole) in 7 ml of water. A solution of 2-chloro-5-(trifluoromethyl)pyridine (9.0 g;

BAD ORIGINAL

0.05 mole) in 40 ml of dimethylsulfoxide was then added to the above sodium phenate solution over an 11 minute period. During the addition, the temperature rose to about 80°C, and then the reaction mixture was heated to 124°C over 26 minutes and the temperature maintained for 15 minutes. At the end of this time, the reaction mixture was cooled to 75°C and poured over ice. The solid product was collected on a filter, washed, and taken up in a toluene-hexane mixture. This solution on cooling yielded 9.7 grams of solid product having a melting temperature of 49.5-50.5°C and having a composition of 58.02% carbon; 3.86% hydrogen; and 5.22% nitrogen. The theoretical composition is 57.99% carbon; 3.74% hydrogen; and 5.20% nitrogen, confirming the product to be 5-(trifluoromethyl)-2-(4-methoxyphenoxy)pyridine.

The 5-(trifluoromethyl)-2-(4-methoxyphenoxy)pyridine (10.95 g; 0.0407 mole) was refluxed with 50 ml of 48 percent by weight aqueous hydrobromic acid solution for one hour. At the end of this time, the reaction mixture was cooled, poured over ice and the separated solids collected on a filter. The product was purified by taking it up in dilute caustic solution, extracting the solution with chloroform to remove unreacted starting material and then acidifying the solution to precipitate free phenol. The dried crystalline phenol product had a melting temperature of 89-91°C and was found to contain 56.21% carbon; 3.27% hydrogen; and 5.44% nitrogen. The theoretical composition of 4-(5-trifluoromethyl-2-pyridyloxy)phenol is 56.48% carbon; 3.16% hydrogen; and 5.49% nitrogen.

The 4-(5-trifluoromethyl-2-pyridyloxy)phenol (4.95 g; 0.0194 mole) was dissolved in dimethylsulfoxide (41 ml); then sodium hydroxide (0.78 g; 0.014 mole) was added as a dry powder and the mixture stirred for about 10 minutes and warmed to about 80°C. Ethyl 2-bromopropionate (4.2 g; 0.023 mole) was then added and the mixture stirred for about 35

minutes at 96°C. The solution was then cooled, poured over ice and the oil, which separated, taken up in petroleum ether containing 20 percent by volume methylene chloride. The separated solvent phase was stripped of solvent leaving an oily product weighing 6.3 g. An infrared scan of a sample of the oil confirmed the ester structure of the anticipated ethyl 2-[4-(5-trifluoromethyl-2-pyridyloxy)phenoxy]propionate.

Ethyl 2-[4-(5-trifluoromethyl-2-pyridyloxy)phenoxy]-propionate (6.3 g; 0.0177 mole) was dissolved in 28 ml of ethanol, and a solution of sodium hydroxide (1.06 g; 0.0266 mole) in 28 ml of water was added. The reaction mixture was heated to 75°C for 5 minutes and then poured into 150 ml of cold water and acidified with 4 g of concentrated hydrochloric acid. The crude acid product, which precipitated, was washed with hot petroleum ether and dried. The resulting product had a melting temperature of 97-100°C and was found on analysis to contain 54.91% carbon; 3.77% hydrogen; and 4.28% nitrogen. The theoretical composition for 2-[4-(5-trifluoromethyl-2-pyridyloxy)phenoxy]propionic acid is 55.05% carbon; 3.70% hydrogen; and 4.28% nitrogen, indicating the expected product was obtained.

Example 2

4-Mercaptophenol (7.6 gm., 0.06 mole) was dissolved in 70 ml of dimethyl sulfoxide, and a solution of sodium hydroxide (2.4 gm., 0.6 mole) in 3.0 ml of water was added. The mixture was warmed to 50° and stirred under nitrogen for 10 minutes to form the sodium thiophenate salt. A solution of 2-chloro-5-(trifluoromethyl)pyridine (10.9 gm., 0.06 mole) in 60 ml of dimethylsulfoxide was then added all at once. The mixture was heated to 100° and held there for 1.5 hours. At the end of this time it was poured into 500 ml of cold water. An emulsion formed; therefore, 60 ml of a saturated solution of ammonium chloride was added. The product

precipitated as a sticky solid. The aqueous layer was decanted, the solid washed with more water then taken up in hot heptane, dried with solid sodium sulfate and decolorized with charcoal. In the filtrate, a white solid product precipitated and was separated and found to have a melting temperature of 89-93°C.

The so-prepared 4-[5-(trifluoromethyl)-2-pyridylthio]phenol (10 gms., 0.037 mole) was dissolved in 80 ml of dimethylsulfoxide, and dry, powdered sodium hydroxide (6.7 gm., 0.37 mole) was added. The mixture was warmed to about 40° and stirred until the base was all in solution indicating that the desired sodium phenate had formed. Ethyl bromopropionate (6.7 gm., 0.37 mole) was then added all at once. The reaction was run at 100-105° for 2.0 hours, then cooled and poured into 450 ml of cold water. The ester was extracted into methylene chloride, the extract dried and solvent removed leaving the product as an oil weighing 13.5 gm.

This was used without further purification for the next step which was hydrolysis of the ester to the metal salt in aqueous alkaline medium.

The ethyl 2-[4-(5-trifluoromethyl-2-pyridylthio)-phenoxy]propionate (13.5 gm., 0.37 mole) was dissolved in 50 ml of 95% ethanol and a solution of sodium hydroxide (3.0 gm., 0.075 mole) in 25 ml of water was added. The mixture was refluxed at 80° for about 6 minutes, then cooled, poured into 400 ml of cold water, and extracted with 250 ml of methylene chloride to remove any base-insoluble impurities. The aqueous solution containing the sodium salt of the acid was acidified to pH 1 with concentrated hydrochloric acid. The product which precipitated as a gummy solid was washed with water (after decanting) and taken up in hot methylcyclohexane. On cooling, the product precipitated as white crystals having a

melting temperature of 118-120°C and a composition of, by weight, 52.38% carbon; 3.66% hydrogen; 4.00% nitrogen and 9.07% sulfur. The theoretical composition of 2-[4-(5-trifluoromethyl-2-pyridylthio)phenoxy]propanoic acid is 52.5% carbon; 3.5% hydrogen; 4.08% nitrogen and 9.34% sulfur.

Example 3

4-Mercaptophenol (6.4 gm., 0.051 mole) was dissolved in 70 ml of dimethylsulfoxide and a solution of sodium hydroxide (2.04 gm., 0.051 mole) in 3.0 ml of water was added. The mixture was warmed to about 50° and stirred under nitrogen for 10 minutes to form the sodium thiophenate salt. A solution of 2,3-dichloro-5-(trifluoromethyl)pyridine (11.0 gm., 0.051 mole) in 60 ml of dimethylsulfoxide was next added all at once. The mixture was then heated at 95-100° for 2.5 hours, then poured into 500 ml of cold water and allowed to stand for 45 minutes. The solid was then collected on a filter, washed and taken up in about one liter of boiling hexane. The product precipitated on cooling as a white solid melting at 94-96°C.

The so-prepared 4-[3-chloro-5-trifluoromethyl-2-pyridylthio]phenol (11.0 gm., 0.036 mole) was dissolved in 80 ml of dimethylsulfoxide and dry powdered sodium hydroxide (1.44 gm., 0.036 mole) was added. The mixture was warmed and stirred until the base was all in solution, showing that the desired sodium phenate had formed. Ethyl bromopropionate (6.5 gm., 0.036 mole) was then added all at once. The reaction was run at 100° for 2.0 hours, then cooled and poured into 500 ml of water. Most of the product precipitated as a white semi-solid. The aqueous layer, which was decanted off was extracted with 300 ml of methylene chloride. The extract was separated, solvent removed, and the residue added to the main product. This way washed thoroughly with water to remove residual dimethylsulfoxide and used without further purification for the hydrolysis.

The so-prepared ethyl 2-[4-(3-chloro-5-trifluoromethyl-2-pyridylthio)phenoxy] propionate (14.6 gm, 0.036 mole) was dissolved in 60 ml of 95% ethanol, and a solution of sodium hydroxide (2.9 gm, 0.072 mole) in 25 ml of water was added. The mixture was heated at reflux for about 4 minutes, then cooled and poured into 400 ml of water, The solution was acidified to pH 1 with concentrated hydrochloric acid which precipitated the product as a sticky solid. This was taken up in a boiling mixture of hexane and methyl cyclohexane. After drying, filtering, and cooling, the white crystalline product separated and was collected on a filter and exhibited a melting temperature of 132-134°C and was found to contain, by weight, 47.64% carbon; 3.14% hydrogen; 3.51% nitrogen; 9.25% chlorine and 8.44% sulfur. The theoretical composition of 2-[4-(3-chloro-5-trifluoromethyl-2-pyridylthio)phenoxy] propanoic acid is 47.69% carbon; 2.93% hydrogen; 3.70% nitrogen; 9.38% chlorine and 8.48% sulfur.

0057473

## CLAIMS

1. A process for preparing trifluoromethylpyridyl (oxy/thio) phenoxypropionic compounds especially active as herbicides for the control of grassy or graminaceous weeds corresponding to the formula I

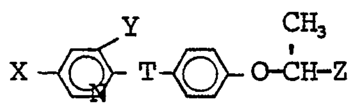

(I)

wherein T is oxygen or sulfur;

X is Cl, Br or $CF_3$;

Y is H, Cl, Br or $CF_3$, provided at least one of X and Y is $CF_3$;

Z is -C-OR, $-CNR_1R_2$, $-C-O^{\ominus}M^{\oplus}$, -CN, $-CH_2OR_3$, $-CH_2OCR_4$, or $-CSR_5$;

M is $^{\oplus}NHR_1R_1R_1$,Na,K,Mg, or Ca;

R is H,$C_{1-8}$ alkyl, benzyl, chlorobenzyl, or $C_{3-6}$ alkoxyalkyl;

$R_1$ is H,$C_{1-4}$ alkyl, or $C_{2-3}$ hydroxyalkyl;

$R_2$ is $R_1$ or $-OCH_3$;

$R_3$ is H or $C_{1-4}$ alkyl;

$R_4$ is $C_{1-7}$ alkyl; and

$R_5$ is $C_{1-4}$ alkyl, as disclosed in the published European Patent application No. 0 000 483 c h a r a c t e r i z e d b y using as an intermediate a trifluoromethylpyridyl(oxy/thio)phenol compound corresponding to the formula II

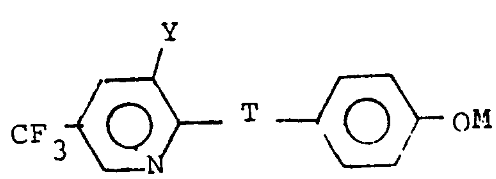

(II)

wherein T is oxygen or sulfur; and

Y is H or Cl, and M is hydrogen or a cation of a caustic solution.

2. The process according to claim 1, c h a r a c t e r i z e d b y using as an intermediate
4-(5-Trifluoromethyl-2-pyridyloxy)phenol,
4-(5-Trifluoromethyl-2-pyridylthio)phenol,
4-(3-Chloro-5-trifluoromethyl-2-pyridylthio)phenol or
4-(3-Chloro-5-trifluoromethyl-2-pyridyloxy)phenol.

3. The process according to claims 1 or 2, c h a r a c t e r i z e d b y reacting said intermediate with a propanoic ester and optionally converting the product obtained to the desired derivative of formula (I).

4. The process according to claim 3, c h a r a c t e r i z e d by dissolving said intermediate in a solvent, adding anhydrous powered sodium hydroxide and reacting therewith, adding the propanoic ester to the reaction mixture, stirring for a time at approximately 100°C and isolating the product obtained.